# EUROPEAN PATENT SPECIFICATION

(11) **EP 0 578 732 B1**
(45) Date of publication and mention of the grant of the patent: **19.06.1996**
(21) Application number: 92909364.9
(22) Date of filing: 13.03.1992
(51) Int. Cl.: A61K 9/00, A61K 9/20

(54) **CHEWABLE ANTACID COMPOSITIONS**
KAUBARE ANTACIDA
COMPOSITIONS ANTACIDES A MACHER

(30) Priority: 04.04.1991 US 680498
(43) Date of publication of application: 19.01.1994
(73) Proprietor: THE PROCTER & GAMBLE COMPANY, Cincinnati, Ohio 45202 (US)
(72) Inventor: UPSON, James Grigg, Springdale, OH 45246 (US); RUSSELL, Carmelita Macklin, Cincinnati, OH 45231 (US); COURT, Paul Randolph, Cincinnati, OH 45231 (US)
(74) Representative: Canonici, Jean-Jacques
(86) International application number: US9201982
(87) International publication number: WO9217161

(56) References cited:
- EP-A- 0 338 861
- EP-A- 0 396 335
- US-A- 4 163 777
- US-A- 4 327 077
- US-A- 4 446 135

## Description

The present invention relates to compressed pharmaceutical compositions in unit dosage forms suitable for ingestion by chewing, said compositions comprising a pre-granulated antacid agent and granulated mannitol, wherein these ingredients are dry blended and direct compressed into a unit dosage form.

Pharmaceutical compositions containing antacid agents useful for treating gastrointestinal disorders are widely used. They vary in the active ingredients, and increasingly differ in the flavors, texture and even forms.

In spite of the large amount of research directed to developing chewable antacid compositions, commercial products to date continue to fail to meet consumer's expectation for an aesthetically acceptable product.

US-A- 4,163,777 discloses antacid compositions in the form of lozenges formulated from a granulation containing antacid, mannitol and a water-insoluble liquid material. There is also mention in this document of the incorporation of gelatin into this granulation. The examples disclose only a wet granulation which has been prepared by blending mannitol with an antacid and adding gelatin solution.

The object of the prior art invention is to provide a product which is specifically intended to dissolve slowly in the mouth [ column 3, line 18].

US-A-4,327,077 discloses a compressed chewable antacid tablet comprising particles of a re-crystallised fatty material and a direct compaction vehicle which can be mannitol and which binds said particles of re-crystallised fatty material and antacid, under compression, into a chewable tablet.

US-A-4,446,135 discloses chewable antacid tablets prepared from a primary granulation of 45-50% by weight of calcium carbonate, 8-11% by weight of magnesium carbonate, 30-40% by weight of sucrose and 8-11% of mannitol, said primary granulation being pressed into chewable tablets together with a flavouring agent and a tablet lubricating agent. It discloses that, by using calcium carbonate having an average particle diameter of from 5-50 µm, good mouth feel is imparted.

EP-A-0,338,861 discloses chewable tablets comprising an antacid blended with granulate mannitol and a hydrophobic substance pressed into tablets, using conventional tableting equipment. The object of the prior art invention is to provide a prolonged gastric residence time.

EP-A-0,396,335 discloses a chewable tablet comprising, for example, antacids and mannitol.

Especially undesirable for the commercial products is the gritty and/or chalky texture and taste/aftertaste present to varying degrees in current products. There continues, therefore, to be a need for antacid products in unit dosage forms suitable for chewing for ingestion which have improved aesthetics. It has been surprisingly discovered that chewable antacid compositions having improved aesthetics can be prepared by a simple, direct compression method of a granulate dry blend when the dry blend comprises granulate mannitol and a pre-granulated antacid agent.

Thus, an object of the present invention is to provide chewable antacid compositions having improved aesthetics. A further object is to provide unit dosage form antacid compositions suitable for ingestion by chewing which have improved texture (e.g., reduce grittiness and/or chalkiness), improve taste and aftertaste, quick disintegration properties, and/or rapid clearance from the mouth, as well as other attributes desirable of a chewable antacid composition. A further object is to provide a simple and efficent process for manufacturing chewable antacid compositions having improved aesthetics, wherein such process involves direct compression of a dry blend of granulate mannitol and pre-granulated antacid agent.

These and other objects of the present invention will become readily apparent from the detailed description which follows.

All percentages and ratios used herein are by weight, and all measurements are made at 25°C, unless otherwise specified.

According to the present invention there are provided compressed compositions in unit dosage form suitable for ingestion by chewing comprising:
(a) pre-granulated antacid agent which has been granulated with gelatin and at least one simple sugar which is a monosaccharide or dissacharide which is safe and effective for ingestion by a human; and
(b) granulated mannitol;
wherein said compositions are prepared by dry blending the pre-granulated antacid agent and the granulated mannitol and direct compressing this blend into a unit dose form.

The present invention also relates to a process for making compressed compositions in unit dosage forms suitable for ingestion by chewing. Said process comprises the steps of: (a) dry blending a composition comprising pre-granulated antacid agent and granulated mannitol; and (b) direct compressing the resulting blend into unit dosage forms suitable for ingestion by chewing.

The present invention compositions comprise a pre-granulated antacid agent as described hereinafter and granulate mannitol compressed into a unit dose form suitable for ingestion by chewing.

Granulate mannitol useful in the compositions of the present invention is commercially available. It is sold, for example, by ICI Americas, Inc., Wilmington, Delaware. Granulate mannitol preferably has a particle size of less than about 1.8mm. Typically, compositions of the present invention comprise from about 25% to about 75% granulate mannitol, preferably from about 30% to about 70%, and most preferably from about 40% to about 60% by weight of the compressed unit dose form.

The term "pre-granulated antacid agent" as used herein, means antacid agents which have been granulated with gelatin and/or simple sugars (e.g., glucose and/or dextrose). The term "antacid agents", as used herein, means not only those ingestible pharmaceutical actives generally recognized as providing benefits by neutralizing stomach acid, but also other ingestible pharmaceutical agents effective for treating the gastrointestinal tract, such as bismuth-containing agents and H₂ receptor-blocking anti-secretory agents, which are preceived as having negative aesthetics improved by compositions according to the present invention. Preferred are antacid agents which have stomach acid neutralizing capacities, such as those agents selected from: aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, and mixtures thereof. Bismuth-containing agents include, for example, bismuth subsalicylate, bismuth aluminate, bismuth citrate, bismuth subcitrate, bismuth nitrate, bismuth subcarbonate, bismuth subgalate, and mixtures thereof. A particularly preferred bismuth salt is bismuth subsalicylate. Examples of H₂ receptor-blocking anti-secretory agents include ranitidine and cimetidine. Preferred antacid agents for use herein are aluminum hydroxide, magnesium hydroxide, dihydroxy aluminum sodium carbonate, calcium carbonate, and mixtures thereof. Most preferred is calcium carbonate.

Pre-granulated antacid agents are commercially available, being sold for example by Wittaker Clark & Daniels, Philadelphia, Pennsylvania. Preferred pre-granulated antacid agents comprise more than about 50%, preferably more than about 75%, and most preferably about 90% or more of antacid agent by weight of the pre-granulate. The pre-granulated antacid agent herein comprises less than about 50% of a granulating agent which is a mixture of gelatin and at least one simple sugar, preferably less than about 25%, and more preferably about 10% or less of granulating agent by weight of the pre-granulate. The term "simple sugars", as used herein, means monosaccharides, disaccharides, and such similar low molecular weight saccharide materials which are safe and effective for ingestion by a human and which are effective for granulated antacid agents when used alone or with gelatin. Most preferred is pre-granulated calcium carbonate comprising from about 80% to about 95% and preferably from about 90% to about 95% calcium carbonate, from about 0.1% to about 5% and preferably from about 0.1% to about 1% gelatin, and from about 1% to about 20% and preferably from about 1% to about 9% glucose. Such pre-granulate is again commercially available from Wittaker Clark & Daniels, Philadelphia, PA.

Typically, the compositions of the present invention comprise from about 25% to about 75% pre-granulated antacid agent by weight of the pharmaceutical composition, preferably from about 30% to about 70%, and most preferably from about 35% to about 50%.

The compositions of the present invention also preferably comprise excipients suitable for use in the present unit dose forms suitable for ingestion by chewing. The term "excipients", as used herein, means one or more compatible solid or liquid filler diluents or encapsulating substances which are suitable for oral administration to a human. The term "compatible", as used herein, means that the components of the compositions of the present invention are capable of being commingled with the antacid active, and with each other, in a manner such that there is no interaction which would substantially reduce the pharmaceutical efficacy of the compositions under ordinary use situations. Excipients must, of course, be of sufficiently high purity and sufficiently low toxicity to render them suitable for administration to the human being treated.

Preferred pharmaceutical compositions of the present invention also comprise as part or all of the excipients an amount of 3-l-menthoxy propane 1,2-diol ("MPD") effective for providing a cooling sensation to the throat. This material is described in detail in US-A-4,459,425, issued July 10, 1984 to Amano et al.. MPD is commercially available from Takasago Perfumery Co., Ltd., Tokyo, Japan.

Some examples of other substances which can serve as excipients are sugars such as lactose, glucose and sucrose; starches such as cornstarch and potato starch; cellulose and its derivatives such as sodium carboxymethylcellulose, ethylcellulose, cellulose acetate; powdered tragacanth; malt; gelatin; talc; stearic acid; magnesium stearate; calcium sulfate; vegetable oils such as peanut oil, cottonseed oil, sesame oil, olive oil, corn oil and oil of theobroma; polyols such as propylene glycol, glycerine, sorbitol, mannitol, and polyethylene glycol; agar; and alginic acid; as well as other non-toxic compatible substances used in pharmaceutical formulations. Wetting agents and lubricants such as sodium lauryl sulfate, as well as coloring agents, flavoring agents, sweetening agents (including non-nutritive sweeteners such as aspartame and saccharin), cooling agents, tableting agents, stabilizers, antioxidants, and preservatives, can also be present. Other compatible pharmaceutical additives and actives (e.g., NSAI drugs; pain killers; muscle relaxants) may be included in the compositions of the present invention. Also, it is to be noted that in addition to the MPD, other materials having cooling properties may also optionally be included within the excipients, such as menthol, and N-ethyl-p-menthane-3-carboxamide ("WS-3", supplied by Sterling Drugs).

The choice of excipients to be used in conjunction with the pharmaceutical active of the present compositions is basically determined by the dose form for the compositions. The preferred dosage forms are compressed chewable tablets comprising a safe and effective amount of the `antacid agent. Excipients suitable for the preparation of such unit dosage forms for oral administration are well-known in the art. Their selection will depend on secondary considerations like taste, cost, shelf stability, which are not critical for the purposes of the present invention, and can be made without difficulty by a person skilled in the art.

The excipients employed in the present compositions are used at concentrations sufficient to provide a practical size to dosage relationship. Typically, excipients comprise from about 1% to about 50% by weight of the pharmaceutical compositions of the present invention, preferably from about 1% to about 40%, and most preferably from about 1% to about 25%. Additionally, the MPD preferably comprises from about 0.01% to about 0.50% by weight of the pharmaceutical compositions of the present invention, preferably from about 0.02% to about 0.20%, and most preferably from about 0.04% to about 0. 10%.

The process for making compositions of the present invention comprised of steps: (a) dry blending a composition comprising pre-granulated antacid agent and granulate mannitol; and (b) direct compressing the resultant blend into unit dosage forms suitable for ingestion by chewing. Preferably, the optional excipients are dry blended with the composition during step (a), but some or all of the excipients may be added as part of the pre-granulated antacid and/or granulated mannitol, or may be added after compression of the composition such as by coating the compressed unit dosage form. Preferred dosage forms are compressed to a hardness of at least about 6 Strong Cobb Units ("S.C."), preferably within from about 6 S.C. to about 15 S.C.

The following examples further describe and demonstrate embodiments within the scope of the present invention.

### Example I

An ingestible pharmaceutical composition according to the present invention in the form of a chewable antacid tablet is prepared as follows:

| Ingredients | Weight % |
|---|---|
| Granulated calcium carbonate¹⁾ | 42.87% |
| Magnesium stearate | 2.50% |
| Colored speckles | 0.75% |
| Flavorants | 0.78% |
| MPD²⁾ | 0.07% |
| WS-3³⁾ | 0.05% |
| Aspartame | 0.198% |
| Sodium Saccharin | 0.102% |
| Mannitol⁴⁾ | Q.S. |

| | |
|---|---|
| 1) Granulated calcium carbonate containing 93.3% calcium carbonate, 6.3% glucose and 0.4% gelatin; supplied by Whittaker Clark & Daniels, Philadelphia, Pa. | |
| 2) 3-l-menthoxy propane 1,2-diol, supplied by Takasago Perfumery Co., Ltd., Tokyo, Japan. | |
| 3) N-ethyl-p-menthane-3-carboxamide, supplied by Sterling Drugs. | |
| 4) Granulate mannitol supplied by ICI Americas, Inc., Wilmington, Delaware. | |

The above ingredients are dry blended in a mixer until homogeneous, and then direct compressed in a tabletting machine to approximately 8.5 S.C. hardness to produce chewable antacid tablets each weighing 1.25g (500mg calcium carbonate per tablet).

Ingestion of one or two of these tablets by a human subject suffering from heartburn, acid indigestion, and upset stomach associated with these symptoms provides effective relief for this upper gastrointestinal tract distress.

## Claims

1. Compressed compositions in unit dosage form suitable for ingestion by chewing comprising:
(a) pre-granulated antacid agent which has been granulated with gelatin and at least one simple sugar which is a monosaccharide or dissacharide which is safe and effective for ingestion by a human; and
(b) granulated mannitol;
wherein said compositions are prepared by dry blending the pre-granulated antacid agent and the granulated mannitol and direct compressing this blend into a unit dose form.

2. Compressed compositions according to Claim 1 wherein the antacid agent present in the pre-granulated antacid agent is selected from antacid agents which have stomach acid neutralizing capacities, bismuth-containing agents, H₂ receptor-blocking anti-secretory agents, and mixtures thereof.

3. Compressed compositions according to either of Claims 1 or 2 wherein the antacid agent is selected from aluminum carbonate, aluminum hydroxide, aluminum phosphate, aluminum hydroxy-carbonate, dihydroxy aluminum sodium carbonate, aluminum magnesium glycinate, dihydroxy aluminum amino acetate, dihydroxy aluminum aminoacetic acid, calcium carbonate, calcium phosphate, aluminum magnesium hydrated sulfates, magnesium aluminate, magnesium alumino silicates, magnesium carbonate, magnesium glycinate, magnesium hydroxide, magnesium oxide, magnesium trisilicate, sucralfate, bismuth subsalicylate, bismuth aluminate, bismuth citrate, bismuth subcitrate, bismuth nitrate, bismuth subcarbonate, bismuth subgalate, ranitidine, cimetidine, and mixtures thereof.

4. Compressed compositions according to any of Claims 1-3 wherein the simple sugar is selected from glucose, dextrose, and mixtures thereof.

5. Compressed compositions in unit dosage form suitable for ingestion by chewing according to Claim 3 comprising:
(a) from 25% to 75% pre-granulated antacid agent; and
(b) from 25% to 75% granulated mannitol.

6. Compressed compositions according to Claim 5 wherein the antacid agent is calcium carbonate and the simple sugar is selected from glucose, dextrose, and mixtures thereof.

7. Compressed chewable antacid tablets comprising:
(a) from 35% to 50% pre-granulated antacid agent comprising from 80% to 95% calcium carbonate, from 0.1% to 5% gelatin, and from 1% to 20% glucose;
(b) from 40% to 60% granulated mannitol; and
(c) from 1% to 25% excipient selected from wetting agents, lubricants, flavoring agent, tableting agents, stabilizers, antioxidants, preservatives, sweetening agents, cooling agents, and mixtures thereof;
and wherein further said compressed tablets are prepared by dry blending the pre-granulated antacid agent and the granulated mannitol and direct compressing this blend to a tablet unit dose form.

8. Compressed chewable antacid tablet compositions according to any of Claims 1-7 comprising from 0.01% to 0.50% 3-l-menthoxy propane 1-2-diol.

9. A process for making a compressed composition according to any of Claims 1-8 comprising the steps of:
(a) dry blending a composition comprising pre-granulated antacid agent and granulated mannitol; and
(b) direct compressing the resulting blend into a unit dosage form suitable for ingestion by chewing.

## Patentansprüche

1. Komprimierte Zusammensetzungen in Einheitsdosisform, welche für die Aufnahme durch Kauen geeignet sind, umfassend:
(a) ein vorgranuliertes Antacidum, welches mit Gelatine und mindestens einem einfachen Zucker, der ein Monosaccharid oder Disaccharid ist, welcher für die Aufnahme durch einen Menschen sicher und wirksam ist, granuliert worden ist: und
(b) granuliertes Mannitol;
wobei die Zusammensetzungen durch Trockenmischen des vorgranulierten Antacidums und des granulierten Mannitols und direktes Komprimieren dieser Mischung zu einer Einheitsdosisform hergestellt sind.

2. Komprimierte Zusammensetzungen nach Anspruch 1, wobei das in dem vorgranulierten Antacidum vorliegende Antacidum aus Antacida, welche Magensäure-Neutralisierungsvermögen aufweisen, Wismut enthaltenden Mitteln, antisekretorischen H₂-Rezeptor-Blockingmitteln und Mischungen hiervon gewählt ist.

3. Komprimierte Zusammensetzungen nach Anspruch 1 oder 2, wobei das Antacidum aus Aluminiumcarbonat, Aluminiumhydroxid, Aluminiumphosphat, Aluminiumhydroxycarbonat, Dihydroxyaluminiumnatriumcarbonat, Aluminiummagnesiumglycinat, Dihydroxyaluminiumaminoacetat, Dihydroxyaluminiumaminoessigsäure, Calciumcarbonat, Calciumphosphat, hydratisierten Aluminiummagnesiumsulfaten, Magnesiumaluminat, Magnesiumaluminosilikaten, Magnesiumcarbonat, Magnesiumglycinat, Magnesiumhydroxid, Magnesiumoxid, Magnesiumtrisilikat, Sucralfat, Wismutsubsalicylat, Wismutaluminat, Wismutzitrat, Wismutsubzitrat, Wismutnitrat, Wismutsubcarbonat, Wismutsubgalat, Ranitidin, Cimetidin und Mischungen hiervon gewählt ist.

4. Komprimierte Zusammensetzungen nach mindestens einem der Ansprüche 1 bis 3, wobei der einfache Zucker aus Glukose, Dextrose und Mischungen hiervon gewählt ist.

5. Komprimierte Zusammensetzungen in Einheitsdosisform, welche für die Aufnahme durch Kauen geeignet sind, nach Anspruch 3, umfassend:
(a) 25 bis 75 % vorgranuliertes Antacidum; und
(b) 25 bis 75 % granuliertes Mannitol.

6. Komprimierte Zusammensetzungen nach Anspruch 5, wobei das Antacidum Calciumcarbonat ist und der einfache Zucker aus Glukose, Dextrose und Mischungen hiervon gewählt ist.

7. Komprimierte kaubare Antacidum-Tabletten, umfassend:
(a) 35 bis 50 % vorgranuliertes Antacidum, das 80 bis 95% Calciumcarbonat, 0.1 bis 5 % Gelatine und 1 bis 20 % Glukose umfaßt;
(b) 40 bis 60 % granuliertes Mannitol; und
(c) 1 bis 25 % eines Arzneimittelträgers, gewählt aus Netzmitteln, Gleitmitteln, Geschmacksmitteln, Tablettierungsmitteln, Stabilisatoren, Antioxidantien, Konservierungsmitteln, Süßungsmitteln, Kühlmitteln und Mischungen hiervon;
und wobei weiterhin die komprimierten Tabletten durch Trockenmischen des vorgranulierten Antacidums und des granulierten Mannitols und direktes Komprimieren dieser Mischung zu einer Tabletten-Einheitsdosisform hergestellt sind.

8. Komprimierte kaubare Antacidum-Tablettenzusammensetzungen nach mindestens einem der Ansprüche 1 bis 7, umfassend 0,01 bis 0,50% 3-l-Menthoxypropan-1-2-diol.

9. Verfahren zur Herstellung einer komprimierten Zusammensetzung nach mindestens einem der Ansprüche 1 bis 8, umfassend die Schritte:
(a) Trockenmischen einer ein vorgranuliertes Antacidum und granuliertes Mannitol umfassenden Zusammensetzung; und
(b) direktes Komprimieren der resultierenden Mischung zu einer zur Aufnahme durch Kauen geeigneten Einheitsdosisform.

## Revendications

1. Compositions de comprimés dans des formes galéniques unitaires adaptées à une ingestion par mastication comprenant :
a) un agent antiacide prégranulé qui a été granulé avec de la gélatine et au moins un sucre simple qui est un monosaccharide ou un disaccharide qui est inoffensif et efficace pour une ingestion par un être humain ; et
b) du mannitol granulé ;
où lesdites compositions sont préparées par mélange à sec de l'agent antiacide prégranulé et du mannitol granulé et par compression directe de ce mélange en une forme galénique unitaire.

2. Compositions de comprimés selon la revendication 1 où l'agent antiacide présent dans l'agent antiacide prégranulé est choisi parmi les agents antiacides qui possèdent des propriétés de neutralisation de l'acidité gastrique, les agents renfermant du bismuth, les agents antisécrétoires bloquant le récepteur d'H₂, et leurs mélanges.

3. Compositions de comprimés selon la revendication 1 ou la revendication 2, où l'agent antiacide est choisi parmi le carbonate d'aluminium, l'hydroxyde d'aluminium, le phosphate d'aluminium, l'hydroxycarbonate d'aluminium, le dihydroxycarbonate d'aluminium et de sodium, le glycinate d'aluminium et de magnésium, l'aminoacétate de dihydroxyaluminium, l'acide dihydroxyaluminium aminoacétique, le carbonate de calcium, le phosphate de calcium, les sulfates d'aluminium et de magnésium hydratés, l'aluminate de magnésium, les aluminosilicates de magnésium, le carbonate de magnésium, le glycinate de magnésium, l'hydroxyde de magnésium, l'oxyde de magnésium, le trisilicate de magnésium, le sucralfate, le salicylate de bismuth basique, l'aluminate de bismuth, le citrate de bismuth, le citrate de bismuth basique, le nitrate de bismuth, le carbonate de bismuth basique, le gallate de bismuth basique, la ranitidine, la cimétidine, et leurs mélanges.

4. Compositions de comprimés selon l'une quelconque des revendications 1-3 où le sucre simple est choisi parmi le glucose, dextrose et leurs mélanges.

5. Compositions de comprimés dans des formes galéniques unitaires appropriées à une ingestion par mastication selon la revendication 3 comprenant :
a) de 25 % à 75 % d'un agent antiacide prégranulé et
b) de 25 % à 75 % de mannitol granulé.

6. Compositions de comprimés selon la revendication 5 où l'agent antiacide est le carbonate de sodium et le sucre simple est choisi parmi le glucose, le dextrose et leurs mélanges.

7. Comprimés antiacides à mâcher comprenant :
a) de 35 % à 50 % d'un agent antiacide prégranulé comprenant du carbonate de calcium à raison de 80 % à 95 %, de la gélatine à raison de 0,1 % à 5 % et du glucose à raison de 1 % à 20 % ;
b) de 40 % à 60 % de mannitol granulé; et
c) de 1 % à 25 % d'un excipient choisi parmi les agents mouillant, les lubrifiants, les aromatisants, les produits entrant dans la fabrication de comprimés, les stabilisateurs, les antioxydants, les conservateurs, les édulcorants, les réfrigérants, et leurs mélanges ;
et où, en outre, lesdits comprimés sont préparés par mélange à sec de l'agent antiacide prégranulé et du mannitol granulé et par compression directe de ce mélange en une forme galénique unitaire comprimée.

8. Composition de comprimés antiacides à mâcher selon l'une quelconque des revendications 1-7 comprenant de 0,01 % à 0,50 % de 3-l-menthoxy propane 1,2-diol.

9. Procédé de préparation d'une composition de comprimés selon l'une quelconque des revendications 1-8 comprenant les étapes de :
a) mélange à sec d'une composition comprenant un agent antiacide prégranulé et du mannitol granulé ; et
b) compression directe du mélange résultant en une forme galénique unitaire appropriée à une ingestion par mastication.
